# EUROPEAN PATENT SPECIFICATION

(11) **EP 3 160 423 B1**
(45) Date of publication and mention of the grant of the patent: **20.12.2017**
(21) Application number: 15720749.9
(22) Date of filing: 12.05.2015
(51) Int. Cl.: A61K 8/25, A61K 8/36, A61K 8/40, A61K 8/49, A61Q 19/00, A61Q 19/08, A61K 8/06, A61K 8/19

(54) **NITROGEN CONTAINING BORANE STABILIZED SKIN CARE COMPOSITIONS**
STICKSTOFF ENTHALTENDE BORANSTABILISIERTE HAUTPFLEGEZUSAMMENSETZUNGEN
AZOTE CONTENANT DES COMPOSITIONS DE SOIN POUR LA PEAU STABILISÉES AU BORANE

(30) Priority: 30.06.2014 EP 14175011
(43) Date of publication of application: 03.05.2017
(73) Proprietor: Unilever PLC, a company registered in England and Wales under company no. 41424 of, London EC4Y 0DY (GB); Unilever N.V., 3013 AL Rotterdam (NL)
(72) Inventor: HUANG, Lei, Trumbull, Connecticut 06611 (US)
(74) Representative: Corsten, Michael Allan
(86) International application number: PCT/EP2015/060509
(87) International publication number: WO 2016/000863

(56) References cited:
- GB-A- 2 181 349
- US-A- 5 534 564
- US-A1- 2005 186 231
- US-B1- 6 287 553

## Description

### Field of the invention

The present invention is directed to skin care compositions containing nitrogen containing borane(s) and conjugated fatty acid(s). The composition unexpectedly results in excellent shelf storage stability.

### Background of the invention

Skin is subject to deterioration through dermatological disorders, environmental abuse (wind, air conditioning, central heating) or through the normal aging process (chronoaging) which may be accelerated by exposure of skin to sun (photoaging). In recent years the demand for cosmetic compositions and cosmetic methods for improving the appearance and condition of skin has grown enormously.

Consumers are increasingly seeking "anti-aging" cosmetic products which treat or delay the visible signs of chronoaging and photoaging skin such as wrinkles, lines, sagging, hyperpigmentation and age spots.

Consumers also frequently seek other benefits from cosmetic products in addition to anti-aging. The concept of "sensitive skin" has also raised the consumer demand for cosmetic products which improve the appearance and condition of sensitive, dry, rough and/or flaky skin and to soothe red, irritated and/or itchy skin. Consumers also desire cosmetic products which treat spots, pimples, blemishes etc.

Skin care cosmetic and dermatological compositions for improving the condition and appearance of skin comprising long chain triglyceride esters of polyunsaturated essential fatty acids, the free acids and their alkali or ammonium salts are well known in the art. For instance, GB 2181349 A describes inter alia a composition composed of triglycerides of linoleic acid for improving the smoothness and elasticity of skin. A commercial product, "Linola Fett n" ex Dr. August Wolff Gmbh, is available for the treatment of dry skin diseases, and dermatoses, which contains inter alia a mixture of the 9,11 isomers of CLA (hereinafter "CLA"). US 6287553 issued to S. Aaluf et. al. on September 11, 2001 describes skin care compositions containing CLA.

There continues to be a need, however, for effective cosmetic compositions for topical application to skin for treating/delaying the visible signs of aging and photo damaged skin such as wrinkles, lines, sagging, hyperpigmentation and age spots that are shelf stable.

We have now surprisingly found that shelf stable compositions that are effective for the treatment and prevention of normal skin conditions due to chronoaging or photoaging, such as wrinkles, lines, sagging, hyperpigmentation and age spots, and/or of sensitive, dry, rough, flaky, red, itchy, irritated skin may be obtained through the application of cosmetic compositions to the skin which contain nitrogen containing borane(s) and conjugated fatty acid(s). Such inventive compositions have reduced formation of malodorous decomposition products and also resist yellowing under the storage conditions described below.

Additionally other benefits were unexpectedly found including that hydrogen released from nitrogen containing borane(s) in the cosmetic composition may create micro-sized bubbles on the order of about 0.1 micron to 100 micron number average diameter to provide a unique tactile sensory quality for such products.

Although not wishing to be bound by the following theory, it is believed that the nitrogen containing borane(s) added to the water phase of skin care product will hydrolyze to release hydrogen gas in-situ. The hydrogen gas will help purge dissolved oxygen in the product to reduce the potential for oxidization.

US 5534564 issued on July 9, 1996 to Y. Zhong et. al. discloses a process for the production of a substantially odorless, color stable N-vinyl heterocyclic copolymer in pure deionized water or in aqueous alcoholic solution which includes contacting an alcohol solution of the copolymer, under reduced pressure, at alcohol reflux temperature with water and a reducing agent selected from the group of a nitrogen containing boranes, sulfurous acid and/or an alkali metal salt of sulfurous acid and recovering an aqueous, 10-80 weight percent solids solution of the copolymer having a substantially color free stability for a period of more than a year.

It is also known to use hydrogen gas in bathing water to improve skin health e.g. the following patent documents: JP 2000-119161 published April 25, 2000; JP 2000-354696 published December 26, 2000; JP 2008-115104 published May 22, 2008 and KR 2009-0056246 published June 3, 2009 all disclose the use of bath water with mixed hydrogen gas or the method for this process. Hydrogen-rich electrolyzed warm water has also been shown to repress wrinkle formation against UVA ray together with type-I collagen production and oxidative-stress diminishment in fibroblasts and cell-injury prevention in keratinocytes. S. Kato et. al. J Photochem Photobiol B. 2012 Jan 5;106:24-33. Epub 2011 Oct 20.

However none of the disclosures cited above describes or suggests a water in oil emulsion cosmetic composition comprising nitrogen containing borane(s) and conjugated fatty acids as claimed in this invention and its unexpected stability within specific limits.

### Summary of the invention

In a first aspect, the present invention is directed to a water in oil emulsion cosmetic composition, including but not limited to:
a. 0.01 to 10 % by wt. of conjugated fatty acid(s);
b. 0.01 to 1 % by wt. of nitrogen containing boranes(s), wherein the weight ratio of the conjugated fatty acid(s) to nitrogen containing boranes(s) is in the weight ratio range of 15 to 5; and
c. 0.5 to 10 % by wt. of emulsifier(s) having an HLB value in the range of 2 to 16.

In a second aspect, the present invention is directed to a method for preparing a stable cosmetic composition containing unsaturated fatty acid(s), including but not limited to the steps of:
a. heating a non-aqueous silicone elastomer, silicone solvent and an emulsifier blend to a temperature in the range of 50-75 °C until all the components are dissolved or well dispersed in a blend;
b. cooling the blend obtained in step (a) to a temperature lower than 35 °C,
c. adding the conjugated fatty acid(s) to the blend with and/or followed by mixing; and
d. adding an aqueous solution of nitrogen containing borane(s) with and/or followed by mixing, until the emulsion is uniform. Preferably the mixing is done at a Low shear rate.

In a third aspect, the present invention is directed to a method of removing malodorous decomposition products from a water in oil emulsion cosmetic composition containing conjugated fatty acid(s) comprising the step of adding a nitrogen containing borane compound; wherein the conjugated fatty acid(s) and nitrogen containing borane(s) are in the weight ratio range of 15 to 5.

All other aspects of the present invention will more readily become apparent upon considering the detailed description and examples which follow.

Skin, as used herein, is meant to include skin on the face, neck, chest, back, arms (including underarms), hands, legs, buttocks and scalp. Active, as used herein, is meant to include a component that improves a skin characteristic and/or benefits skin wherein the same can be, and preferably, is an active in a leave-on composition, and most preferably, a cream, lotion, balm, deodorant, or gel.

Comprising, as used herein, is meant to include consisting essentially of and consisting of. For the avoidance of doubt, therefore, the skin care composition and method of this invention may consist essentially of or consist of nitrogen containing borane(s) and conjugated fatty acid(s). Emulsion means water-in-oil emulsion and may be interchanged with composition of this invention, where the composition of this invention is often one that is employed to evenly deposit active which is/are a conjugated fatty acid(s). All ranges identified herein are meant to include all ranges subsumed therein if, for example, reference to the same is not explicitly made.

Except in the operating and comparative examples, or where otherwise explicitly indicated, all numbers in this description indicating amounts or ratios of materials or conditions or reaction, physical properties of materials and/or use are to be understood as modified by the word "about".

Where used in the specification, the term "comprising" is intended to include the presence of stated features, integers, steps, components, but not to preclude the presence or addition of one or more features, integers, steps, components or groups thereof.

All percentages in the specification and examples are intended to be by weight unless stated otherwise.

### Detailed description of the preferred embodiments

In a first aspect, the present invention is directed to a water in oil emulsion cosmetic composition, including but not limited to:
a. 0.01 to 10 % by wt. of conjugated fatty acid(s) (preferably conjugated octadeca and/or eicosa dienoic and/or trienoic fatty acid(s) such as CLA and other conjugated acids and isomers thereof useful for skin treatment); preferably the acid(s) present at a minimum concentration of 0.01, 0.05, or 0.1 % by wt. and a maximum concentration of 1, 5, or 10 % by wt.
b. 0.01 to 1 % by wt. of nitrogen containing boranes(s) (preferably including borane ammonia); preferably present at a minimum concentration of 0.01, 0.02, or 0.05 % by wt. and a maximum concentration of 0.1, 0.5, or 1 % by wt., wherein the weight ratio of the conjugated fatty acid(s) to nitrogen containing boranes(s) is in the range of 15 to 5; preferably 12 to 8; and
c. 0.5 to 10 % by wt. of emulsifier(s) having an HLB value in the range of 2 to 16; preferably selected from the group of Caprylic/Capric Triglycerides, Stearic Acid, Cetyl Alcohol, Sucrose Distearate, PEG-10 Dimethicone and blends thereof and the like; preferably the emulsifier(s) are present at a minimum concentration of 0.5 or 1 % by wt. and a maximum concentration of 1, 5 or 10% by wt.

It was found that such compositions show an unexpected stability. The compositions of the present invention are thus stable to decomposition.

Advantageously the inventive composition further includes 10 to 70 % by wt. of silicone elastomer(s) or a blend thereof; preferably present at a minimum concentration of 10, 20, or 25 % by wt. and a maximum concentration of 30, 50, or 70 % by wt.

Advantageously the inventive composition further includes 5 to 20 % by wt. of silicone solvent(s) that are capable of swelling the silicone elastomer(s) and that are liquid or flowable at 25 °C; preferably present at a minimum concentration of 5, or 10 % by wt. and a maximum concentration of 15, or 20 % by wt.

Preferably the inventive composition includes 0.01 to 10 % by wt. of water soluble and/or dispersible skin actives, preferably alpha hydroxy acid(s) and/or vitamins and the like; the active(s) preferably present at a minimum concentration of 2, or 3 % by wt. and a maximum concentration of 4 or 10% by wt.

Preferably the inventive composition includes 0.01 to 10 % by wt. of polyhydric alcohol(s) such as glycerin, propylene glycol, butane diol, blends thereof and the like, that are liquid or flowable at 25 °C; preferably the alcohol(s) are present at a minimum concentration of 0.1, or 0.5 % by wt. and a maximum concentration of 1, or 10% by wt.

In another aspect of the invention is a method for preparing a stable cosmetic composition containing unsaturated fatty acid(s), including but not limited to the steps of:
a. heating a non-aqueous silicone elastomer, silicone solvent and emulsifier blend to a temperature in the range of 50-75 °C until all the components are dissolved or well dispersed in a blend;
b. cooling the blend produced in step (a) to a temperature lower than 35 °C,
c. adding the conjugated fatty acid(s) (and optionally other water soluble skin treatment actives) to the blend with and/or followed by mixing, preferably at a Low shear rate; and
d. adding an aqueous solution of nitrogen containing borane(s) (preferably including borane ammonia) with and/or followed by mixing, preferably at a Low shear rate (as that term is defined below) until the emulsion is uniform.

Advantageously the inventive method includes the step of adding an aqueous phase containing neutralized alpha hydroxy acid(s), vitamins and/or other water soluble and/or dispersible skin treatment actives to the non-aqueous silicone elastomer, silicone solvent and emulsifier blend prepared in step (a) with and/or followed by mixing until the resulting emulsion is uniform and homogenous.

Preferably the mixing is done at a High shear rate (as that term is defined below), preferably at a temperature of 50 to 60 °C.

Low shear rate is herein defined as the shear produced that is equivalent to a Heidolph RZR 2052 mixer (available from Heidolph North America, Elk Grove Village, IL) equipped with a BR 10 Crossed Blade Impeller operated at a speed of 200 rpm or less and High shear rate is herein defined as the shear produced that is equivalent to the same mixer and blade operated at a speed of 750 rpm or more.

In another aspect of the invention is a method of removing malodorous decomposition products from a water in oil emulsion cosmetic composition containing conjugated fatty acid(s) comprising the step of adding a nitrogen containing borane compound (preferably including borane ammonia); wherein the conjugated fatty acid(s) and nitrogen containing borane(s) are in the weight ratio range of 15 to 1 and more preferably wherein the cosmetic composition includes:
a. 0.01 to 10 % by wt. of conjugated fatty acid(s) (preferably conjugated octadeca and/or eicosa dienoic and/or trienoic fatty acid(s) such as CLA and other similar acids and isomers useful for skin treatment; preferably the acid(s) are present at a minimum concentration of 0.01, 0.05, or 0.1 % by wt. and a maximum concentration of 1,5, or 10 % by wt.
b. 0.01 to 1 % by wt. of nitrogen containing boranes(s) (preferably including borane ammonia); preferably present at a minimum concentration of 0.01, 0.02, or 0.05 % by wt. and a maximum concentration of 0.1, 0.5, or 1 % by wt., wherein the weight ratio of the conjugated fatty acid(s) to nitrogen containing boranes(s) is in the range of 15 to 5; preferably 12 to 8; and
c. 0.5 to 10 % by wt. of emulsifier(s) having an HLB value in the range of 2 to 16; preferably selected from the group of Caprylic/Capric Triglycerides, Stearic Acid, Cetyl Alcohol, Sucrose Distearate, PEG-10 Dimethicone and blends thereof and the like; preferably the emulsifier(s) are present at a minimum concentration of 0.5 or 1 % by wt. and a maximum concentration of 1, 5 or 10% by wt.

In a preferred embodiment, the addition of the nitrogen containing borane compound is made after the composition has been stored at least for 30 days and preferably at or above ambient temperature of 20 or 25 degrees C or at or above an elevated temperature of 30, 35, 40 or 45 °C.

The nitrogen containing borane(s) of this invention include organic or inorganic nitrogen containing compounds such as borane ammonia (BH₃NH₃); morpholine borane optionally substituted with a lower (C₁ to C₄) alkyl group, e.g. 4-ethyl morpholine borane; borane C₁ to C₄ alkyl amine, e.g. borane t-butyl amine ([CH₃]₃ C-NH₂BH₃), borane trimethyl amine; tetramethyl ammonium octahydro triborate and a silicon containing borane, e.g. silylamino borane (SiH₃ --BH₂ --NH₃); blends thereof and the like.

The conjugated fatty acids employable herein desirably comprise one or more of oleic acid, linoleic acid, linolenic acid or ricinoleic acid. Various isomers of such acids often have common names, including linolenelaidic acid, trans 7-octadecenoic acid, parinaric acid, pinolenic acid punicic acid, petroselenic acid and stearidonic acid. It is especially desirable to employ fatty acids selected from oleic acid, linoleic acid or petroselenic acid, or a mixture containing one or more of them.

In a preferred embodiment the conjugated fatty acid used in the invention includes CLA or a derivative thereof as discussed below. Preferably at least 10, 20, 30, 40, 50, 60, 70, 80, 90, or 95 % by wt. or more of the conjugated fatty acid is CLA and/or a derivative thereof. CLA comprises a group of positional and geometric isomers of linoleic acid in which various configurations of cis and trans double bonds at positions (6, 8), (7, 9), (8, 10), (9, 11), (10, 12) or (11, 13) are possible. Thus twenty-four different isomers of CLA exist.

A more preferred active of the composition in accordance with the present invention is the c9, trans 11 (hereinafter referred to as c9, t11) isomer. This particular isomer of the free acid has the structure (I) shown below:

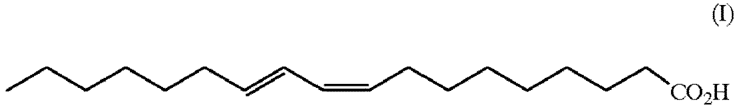

As discussed above, the invention also includes derivatives of the free acid which thus comprise CLA moieties. Preferable derivatives include those derived from substitution of the carboxyl group of the acid, such as esters (e.g. retinyl esters, triglyceride esters, monoglyceride esters, diglyceride esters, phosphoesters), amides (e.g. ceramide derivatives), salts (e.g. alkali metal and alkali earth metal salts, ammonium salts); and/or those derived from substitution of the C18 carbon chain, such as alpha hydroxy and/or beta hydroxy derivatives.

In the case of triglyceride ester derivatives, all positional isomers of CLA substituents on the glycerol backbone are included. The triglycerides preferably contain at least one CLA moiety. For example, of the three esterifiable positions on the glycerol backbone, the 1 and 2 positions may be esterified with CLA and by another lipid at position 3 or as an alternative; the glycerol backbone could be esterified by CLA at the 1 and 3 positions with another lipid at position 2.

Wherever the term CLA is used in this specification it is to be understood that the derivatives thereof comprising CLA moieties are also included. "CLA moieties" refers to CLA fatty acyl portion(s) of a CLA derivative.

By "c9 t11 isomer enriched CLA" is meant that at least 50% by weight of the total CLA (and/or CLA) moieties present in the composition is in the form of the cis 9, trans 11 isomer. Preferably, at least 70%, most preferably at least 90%, by weight of the total CLA and/or CLA moieties present in the composition, is in the form of the c9, t11 isomer.

The CLA and/or derivatives thereof comprising CLA moieties according to the present invention (in which preferably at least 50% by weight of the total CLA and/or CLA moieties present in the composition is in the form of the cis 9, trans 11 isomer) may be prepared according to the method disclosed in PCT publication no. WO 97/18320 published on 22 May 1997.

The active, c9 t11 isomer enriched CLA, to preferably employed in accordance with the present invention is present in the topical composition in an effective amount. Normally the total amount of the active is present in an amount between 0.00001% and 50% by weight of the composition. More preferably the amount is from 0.01% to 10% and most preferably from 0.1% to 5% in order to maximise benefits at a minimum cost.

Actives suitable for use in this invention are meant to include but not be limited to opacifiers, colorants, skin lightening agents, moisturizers, sunscreens, plant extracts, anti-inflammatories, surfactants, wrinkle reducing agents, mixtures thereof or the like.

Compositions (or oil-in-water emulsions) of the present invention may typically include cosmetically acceptable carrier components in addition to the water, oil, nitrogen containing borane(s) and conjugated fatty acids described herein. Water, nevertheless, is the most preferred carrier. Amounts of water may range from 1 to 98%, and preferably, from 5 to 90%, and most preferably, from 35 to 80%, and optimally, from 40 to 75% by weight, based on total weight of the composition and including all ranges subsumed therein.

Cosmetically acceptable carriers suitable for use in this invention may include mineral oils, silicone oils, synthetic or natural esters, and alcohols. Amounts of these materials may range from 0.1 to 50%, and preferably, from 0.1 to 30%, and most preferably, from 1 to 20% by weight of the composition, including all ranges subsumed therein.

Silicone oils may be divided into the volatile and non-volatile variety. The term "volatile" as used herein refers to those materials which have a measurable vapor pressure at ambient temperature. Volatile silicone oils are preferably chosen from cyclic or linear polydimethylsiloxanes containing from 3 to 9, and preferably, from 4 to 5 silicon atoms.

Linear volatile silicone materials generally have viscosities of less than 5 centistokes at 25 °C while cyclic materials typically have viscosities of less than 10 centistokes.

Nonvolatile silicone oils useful as carrier material include polyalkyl siloxanes, polyalkylaryl siloxanes and polyether siloxane copolymers. The essentially non-volatile polyalkyl siloxanes useful herein include, for example, polydimethylsiloxanes (like dimethicone) with viscosities of from 5 to 100,000 centistokes at 25 °C. Silicone oils (especially, Dimethicone 35 to 75 centistokes) suitable for use are often made commercially available from Dow Corning are preferred.

Among suitable esters are:
(1) Alkenyl or alkyl esters of fatty acids having 10 to 20 carbon atoms like isopropyl palmitate, isopropyl isostearate, isononyl isonanonoate, oleyl myristate, isopropyl myristate, oleyl stearate, and oleyl oleate;
(2) Ether-esters such as fatty acid esters of ethoxylated fatty alcohols;
(3) Polyhydric alcohol esters such as ethylene glycol mono- and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters;
(4) Wax esters such as beeswax, spermaceti, myristyl myristate, stearyl stearate; and
(5) Sterol esters, of which soya sterol and cholesterol fatty acid esters are examples thereof.

Emulsifiers are preferably present in the composition of the present invention. Total concentration of the emulsifier may range from 0.1 to 40%, and preferably, from 1 to 20%, and most preferably, from 1 to 5% by weight of the composition, including all ranges subsumed therein. The emulsifier may be selected from the group consisting of anionic, nonionic, cationic and amphoteric actives. Particularly preferred nonionic actives are those with a C₁₀-C₂₀ fatty alcohol or acid hydrophobe condensed with from 2 to moles of ethylene oxide or propylene oxide per mole of hydrophobe; C₂-C₁₀ alkyl phenols condensed with from 2 to 20 moles of alkylene oxide; mono- and di- fatty acid esters of ethylene glycol; fatty acid monoglyceride; sorbitan, mono- and di- C₈-C₂₀ fatty acids; and polyoxyethylene sorbitan as well as combinations thereof. Alkyl polyglycosides and saccharide fatty amides (e.g. methyl gluconamides) are also suitable nonionic emulsifiers.

Preferred anionic emulsifiers include alkyl ether sulfate and sulfonates, alkyl sulfates and sulfonates, alkylbenzene sulfonates, alkyl and dialkyl sulfosuccinates, C₈-C₂₀ acyl isethionates, C₈-C₂₀ alkyl ether phosphates, alkylethercarboxylates and combinations thereof.

Cationic emulsifiers that may be used include, for example, palmitamidopropyltrimonium chloride, distearyldimonium chloride and mixtures thereof. Useful amphoteric emulsifiers include cocoamidopropyl betaine, C₁₂-C₂₀ trialkyl betaines, sodium lauroamphoacetate, and sodium laurodiamphoacetate or a mixture thereof.

Other generally preferred emulsifiers include glyceryl stearate, glycol stearate, stearamide AMP, PEG-100 stearate, cetyl alcohol as well as emulsifying/thickening additives like hydroxyethylacrylate/sodium acryloyldimethyl taurates copolymer/squalane and mixtures thereof.

Emulsion stabilizers generally classified as vegetable based liquids may also be used. Preferred stabilizers are sold under the name Oilwax LC and made available commercially by Lotioncrafter.

In addition to the nitrogen containing borane(s) preservatives of the invention additional preservatives can desirably be incorporated into the compositions of this invention to protect against the growth of potentially harmful microorganisms. Suitable traditional preservatives for compositions of this invention are alkyl esters of para-hydroxybenzoic acid. Other preservatives which have more recently come into use include hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are iodopropynyl butyl carbamate, phenoxyethanol, methyl paraben, propyl paraben, imidazolidinyl urea, sodium dehydroacetate and benzyl alcohol. The preservatives should be selected having regard for the use of the composition and possible incompatibilities between the preservatives and other ingredients in the emulsion. Additional preservatives are preferably employed in amounts ranging from 0.01% to 2% by weight of the composition, including all ranges subsumed therein.

Thickening agents may optionally be included in compositions of the present invention. Particularly useful are the polysaccharides. Examples include starches, natural/synthetic gums and cellulosics. Representative of the starches are chemically modified starches such as sodium hydroxypropyl starch phosphate and aluminum starch octenylsuccinate. Tapioca starch is often preferred. Suitable gums include xanthan, sclerotium, pectin, karaya, arabic, agar, guar, carrageenan, alginate and combinations thereof. Suitable cellulosics include hydroxypropyl cellulose, hydroxypropyl methylcellulose, ethylcellulose and sodium carboxy methylcellulose. Synthetic polymers are yet another class of effective thickening agent. This category includes crosslinked polyacrylates such as the Carbomers, polyacrylamides such as Sepigel® 305 and taurate copolymers such as Simulgel EG® and Aristoflex® AVC, the copolymers being identified by respective INCI nomenclature as Sodium Acrylate/Sodium Acryloyldimethyl Taurate and Acryloyl Dimethyltaurate/Vinyl Pyrrolidone Copolymer. Another preferred synthetic polymer suitable for thickening is an acrylate-based polymer made commercially available by Seppic and sold under the name Simulgel INS100.

Amounts of the thickener, when used, may range from 0.001 to 5%, and preferably, from 0.1 to 3%, and most preferably, from 0.2 to 1.5% by weight of the composition including all ranges subsumed therein.

Conventional humectants may be employed in the present invention. These are generally polyhydric alcohol-type materials. Typical polyhydric alcohols include glycerol (i.e., glycerine or glycerin), propylene glycol, dipropylene glycol, polypropylene glycol, polyethylene glycol, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. Most preferred is glycerin, propylene glycol or a mixture thereof. The amount of humectant employed may range anywhere from 0.5 to 20%, preferably between 1 and 15% by weight of the composition.

Fragrances, colorants, fixatives and abrasives may optionally be included in compositions of the present invention. Each of these substances may range from 0.05 to 5%, preferably between 0.1 and 3% by weight.

Turning to the actives suitable for use herein, the same can include opacifiers like TiO₂ and ZnO and colorants like iron oxide red, yellow and black. Such opacifiers and colorants typically have a particle size from 50 to 1200 nm, and preferably, from 50 to 350 nm.

To enhance skin moisturization, actives classified as cationic ammonium compounds may optionally be used in the compositions of this invention. Such compounds include salts of hydroxypropyltri (C₁-C₃ alkyl) ammonium mono-substituted-saccharide, salts of hydroxypropyltri (C₁-C₃ alkyl) ammonium mono-substituted polyols, dihydroxypropyltri (C₁-C₃ alkyl) ammonium salts, dihydroxypropyldi (C₁-C₃ alkyl) mono(hydroxyethyl) ammonium salts, guar hydroxypropyl trimonium salts, 2,3-dihydroxypropyl tri(C₁-C₃ alkyl or hydroxalkyl) ammonium salts or mixtures thereof. In a most preferred embodiment and when desired, the cationic ammonium compound employed in this invention is the quaternary ammonium compound 1,2-dihydroxypropyltrimonium chloride. If used, such compounds typically make up from 0.01 to 30%, and preferably, from 0.1 to 15% by weight of the composition.

When cationic ammonium compounds are used, preferred additional active for use with the same are moisturizing agents such as substituted ureas like hydroxymethyl urea, hydroxyethyl urea, hydroxypropyl urea; bis(hydroxymethyl) urea; bis(hydroxyethyl) urea; bis(hydroxypropyl) urea; N,N'-dihydroxymethyl urea; N,N'-di-hydroxyethyl urea; N,N'-di-hydroxypropyl urea; N,N,N'-tri-hydroxyethyl urea; tetra(hydroxymethyl) urea; tetra(hydroxyethyl) urea; tetra(hydroxypropyl) urea; N-methyl-N'-hydroxyethyl urea; N-ethyl-N,N-N'-hydroxyethyl urea; N-hydroxypropyl-N'-hydroxyethyl urea and N,N'-dimethyl-N-hydroxyethyl urea or mixtures thereof. Where the term hydroxypropyl appears, the meaning is generic for either 3-hydroxy-n-propyl, 2-hydroxy-n-propyl, 3-hydroxy-i-propyl or 2-hydroxy-i-propyl radicals. Most preferred is hydroxyethyl urea. The latter is available as a 50% aqueous liquid from the National Starch & Chemical Division of ICI under the trademark Hydrovance.

Amounts of substituted urea, when used, in the composition of this invention range from 0.01 to 20%, and preferably, from 0.5 to 15%, and most preferably, from 2 to 10% based on total weight of the composition and including all ranges subsumed therein.

When cationic ammonium compound and substituted urea are used, in a most especially preferred embodiment at least from 1 to 15% glycerin external to the particle is used, based on total weight of the composition and including all ranges subsumed therein.

Compositions of the present invention may include vitamins as the desired active. Illustrative vitamins are Vitamin A (retinol) as well as retinol esters like retinol palmitate and retinol propionate, Vitamin B₂, Vitamin B₃ (niacinamide), Vitamin B₆, Vitamin C, Vitamin E, Folic Acid and Biotin. Derivatives of the vitamins may also be employed. For instance, Vitamin C derivatives include ascorbyl tetraisopalmitate, magnesium ascorbyl phosphate and ascorbyl glycoside. Derivatives of Vitamin E include tocopheryl acetate, tocopheryl palmitate and tocopheryl linoleate. DL-panthenol and derivatives may also be employed. Total amount of vitamins when present in compositions according to the present invention may range from 0.001 to 10%, preferably from 0.01% to 1%, optimally from 0.1 to 0.5% by weight of the composition.

Azelaic acid, ubiquinone, dihydroxyacetone (DHA) and mixtures thereof may also be used as actives in the composition of this invention. Such compounds, when used, typically make up from 0.2 to 4.5%, and preferably, from 0.5 to 3% by weight of the composition, including all ranges subsumed therein.

Other optional actives suitable for use in this invention include resveratrol, resorcinols like 4-ethyl resorcinol, 4-hexyl resorcinol, 4-phenylethyl resorcinol, dimethoxytoluyl propyl resorcinol, 4-cyclopentyl resorcinol, 4-cyclohexylresorcinol, alpha-an/or beta-hydroxyacids, phenylethyl resorcinol (Symwhite 377 from Symrise), undecylenol phenylalanine (Seppi White from Seppic) mixtures thereof or the like. Such actives, when used, collectively make up from 0.001 to 12% by weight of the composition.

Desquamation promoters may be present. Illustrative are the alpha-hydroxycarboxylic acids, beta-hydroxycarboxylic acids. The term "acid" is meant to include not only the free acid but also salts and C₁-C₃₀ alkyl or aryl esters thereof and lactones generated from removal of water to form cyclic or linear lactone structures. Representative acids are glycolic and its derivatives, lactic and malic acids. Salicylic acid is representative of the beta-hydroxycarboxylic acids. Amounts of these materials when present may range from 0.01 to 15% by weight of the composition.

A variety of herbal extracts may optionally be included as actives in compositions of this invention. The extracts may either be water soluble or water-insoluble carried in a solvent which respectively is hydrophilic or hydrophobic. Water and ethanol are the preferred extract solvents. Illustrative extracts include those from green tea, yarrow, chamomile, licorice, aloe vera, grape seed, citrus unshui, willow bark, sage, thyme and rosemary. Soy extracts may be used and especially when it is desirable to include retinol.

Also optionally suitable for use include materials like chelators (e.g., EDTA), C₈₋₂₂ fatty acid substituted saccharides, lipoic acid, retinoxytrimethylsilane (available from Clariant Corp. under the Silcare 1M-75 trademark), dehydroepiandrosterone (DHEA) and combinations thereof. Ceramides (including Ceramide 1, Ceramide 3, Ceramide 3B and Ceramide 6) as well as pseudoceramides may also be useful. Occlusives like Oilwax LC are often desired. Amounts of these materials may range from 0.000001 to 10%, preferably from 0.0001 to 1% by weight of the composition.

Sunscreen actives may also be included in compositions of the present invention and carried by the particle comprising hydrophobic material as described herein. Particularly preferred are such materials as phenylbenzimidazole sulfonic acid (Ensulizole), ethylhexyl p-methoxycinnamate, available as Parsol MCX®, Avobenzene, available as Parsol 1789® and benzophenone-3, also known as Oxybenzone. Inorganic sunscreen actives may be employed such as microfine titanium dioxide, zinc oxide, polyethylene and various other polymers. Also suitable for use is octocrylene. Amounts of the sunscreen agents when present may generally range from 0.1 to 30%, preferably from 0.5 to 20%, optimally from 0.75 to 10% by weight.

Conventional buffers/pH modifiers may be used. These include commonly employed additives like sodium hydroxide, potassium hydroxide, hydrochloric acid, citric acid and citrate/citric acid buffers. In an especially preferred embodiment, the pH of the composition of this invention is from 4 to 8, and preferably, from 4.25 to 7.75, and most preferably, from 6 to 7.5, including all ranges subsumed therein. The composition of this invention may be a solid stick or bar. Viscosity of the composition of this invention is, however, preferably from 1,000 to 120,000 cps, and most preferably, from 5,000 to 80,000 cps, taken at ambient temperature NS and a shear rate of 1s⁻¹ with a strain controlled parallel plate rheometer made commercially available from suppliers like T.A. Instruments under the Ares name.

A wide variety of packaging can be employed to store and deliver the composition of this invention. Preferably the package should be able to contain or prevent any elevated pressure build-up during storage and use of the product. Pump dispensers configured to either prevent or withstand high pressure build-up, may be used.

Packaging is often dependent upon the type of personal care end-use. For instance, leave-on skin lotions and creams, shampoos, conditioners and shower gels generally employ plastic containers with an opening at a dispensing end covered by a closure. Typical closures are screw-caps, non-aerosol pumps and flip-top hinged lids. Packaging for antiperspirants, deodorants and depilatories may involve a container with a roll-on ball on a dispensing end. Alternatively these types of personal care products may be delivered in a stick composition formulation in a container with propel-repel mechanism where the stick moves on a platform towards a dispensing orifice. Metallic cans pressurized by a propellant and having a spray nozzle serve as packaging for antiperspirants, shave creams and other personal care products. Toilette bars may have packaging constituted by a cellulosic or plastic wrapper or within a cardboard box or even encompassed by a shrink wrap plastic film.

The following examples are provided to facilitate an understanding of the present invention. The examples are not intended to limit the scope of the claims.

### Examples

### Example 1

The cosmetic water in oil emulsion composition containing CLA described in Table 1 was prepared according to the procedure below in order to ascertain the effect of concentration of borane ammonia on malodor decomposition products produced under accelerated storage conditions. Results are presented in Table 2.

**TABLE 1**

| **INGREDIENT NAME** | **% w/w** |
|---|---|
| ***PHASE A*** | |
| Caprylic/Capric Triglyceride | 1.000 |
| Cyclopentasiloxane (and) Dimethicone Crosspolymer (Dow Corning 9045) | 50.000 |

| ***PHASE B*** | |
|---|---|
| Caprylic/Capric Triglyceride | 0.530 |
| Stearic Acid | 0.200 |
| Cetyl Alcohol | 0.200 |
| Sucrose Distearate | 0.100 |
| Cyclopentasiloxane (DC 245 fluid, 99% Active) | 10.907 |
| Dodecamethyl cyclohexasiloxane (DC 246 fluid) | 5.835 |
| Butylated hydroxytoluene | 0.200 |

| ***PHASE C*** | |
|---|---|
| PEG-10 Dimethicone | 0.900 |
| Titanium Dioxide | 0.250 |

| ***PHASE D*** | |
|---|---|
| Water, deionized | 4.273 |
| Amino tri (methylene phosphonic acid) pentasodium salt (Dequest 2006) | 0.490 |
| *DMDM Hydantoin (and) lodopropynyl Butylcarbamate* (Glydant Plus Liquid) | 0.200 |
| Potassium Hydroxide (45%) | 5.535 |
| Glycolic Acid (70% solution) | 5.710 |
| Glycerin | 10.000 |

| ***PHASE E*** | |
|---|---|
| Retinol | 0.1 |
| Conjugated linoleic acid (CLA) | 1.000 |

| **PHASE F** | |
|---|---|
| Water, deionized | 2.000 |
| borane ammonia | 0-1 |
| **TOTAL** | 100.000 |

### Sample preparation procedure:

Heat up Phase A in main mixer to 50-55 °C and mix it at Low shear rate until it is uniform. Heat up phase B to 70-75 °C, mix until uniform, then cool to 55 °C. Add phase B to phase A in main mixer at 50-55 °C. Pre-wet Titanium Dioxide with PEG-10 Dimethicone (phase C). Add phase C to phase A and B and mix all ingredients in main mixer at Low shear rate of 200 rpm to make sure all ingredients are well dispersed in a blend. Keep the mixed phases at 50-55 °C. Dissolve all ingredients in phase D and heat to 55 °C. Increase shear rate in main mixer to a high shear rate of about 850 rpm and add phase D to main mixer slowly at this shear rate. Continue to mix at a high shear rate for 5 minutes. Homogenize the batch for another 3 minutes to make sure the emulsion is smooth and uniform. Then slow down the shearing in the main mixer and start to cool down the batch. When main batch temperature is lower than 35 °C, add phase E to main mixer at Low shear rate. Dissolve required percentage of borane ammonia in water and then add phase F to main mixer at Low shear rate until blend becomes uniform.

All samples prepared with a are packed in 4oz glass jars leaving an air space of approx. 1 cm high, tightly covered with screw caps and stored in an oven at 45 °C for one month in the dark. The odor of the samples was evaluated by 5 experienced lab workers in random order. The relative odor scale is defined as the range from -5 to 5, where -5 means highly objectionable odor, 0 means neutral odor and 5 means pleasant odor. The evaluated smell scores were recorded and the average smell scores for different samples are shown in table 2.

### Odor scale:

| | | | | |
|---|---|---|---|---|
| -5 | 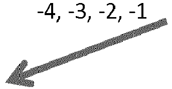 | 0 | 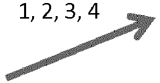 | 5 |
| awful | | neutral | | pleasuring |

**TABLE 2, Effect on odor when borane ammonia is added prior to storage**

| Level of borane ammonia in product wt. %, | odor scale after storage | Weight ratio of CLA to borane ammonia |
|---|---|---|
| 0 | -3 | |
| 0.05 | -2 | 50 |
| 0.1 | 0 | 10 |
| 0.5 | -4 | 2 |

### Example 2

The cosmetic water in oil emulsion composition containing CLA described in Table 1 was prepared according to the sample preparation procedure described above in order to ascertain the effect of post-adding of borane ammonia on malodor decomposition products after storage at elevated temperature. In this test, prototypes without any borane ammonia were prepared and stored in 45 °C for one month. Then, different levels of borane ammonia were post-added to the aged samples. The odor evaluations were conducted after 1 hour of adding borane ammonia while the samples were held in sealed jars at 25 °C. The results are presented in table 3.

**TABLE 3**

| Level of borane ammonia %, (Post added after storage) | Weight ratio of CLA to borane ammonia | odor scale before adding borane ammonia | odor scale after adding borane ammonia |
|---|---|---|---|
| 0 | | -3 | -3 |
| 0.05 | 50 | -3 | -1 |
| 0.1 | 10 | -3 | 2 |
| 0.5 | 2 | -3 | 2 |

## Claims

1. A water in oil emulsion cosmetic composition, comprising:
a. about 0.01 to 10 % by wt. of conjugated fatty acid(s);
b. about 0.01 to 1 % by wt. of nitrogen containing boranes(s), wherein the weight ratio of the conjugated fatty acid(s) to nitrogen containing boranes(s) is in the range of about 15 to 5; and
c. about 0.5 to 10 % by wt. of emulsifier(s) having an HLB value in the range of about 2 to 16.

2. The composition of claim 1 wherein at least 10 % by wt. of the conjugated fatty acid(s) is CLA (conjugated linoleic acid) and/or a derivative thereof.

3. The composition of claim 1 further comprising about 10 to 70 % by wt. of silicone elastomer(s) or a blend thereof.

4. The composition of claim 2 further comprising about 5 to 20 % by wt. of silicone solvent(s) that are capable of swelling the silicone elastomer(s) wherein the solvent(s) are liquid or flowable at 25 C.

5. The composition of claim 1 further comprising about 0.01 to 10 % by wt. of water soluble or dispersible skin actives.

6. The composition of claim 5 wherein the skin actives include alpha hydroxy acid(s) and/or vitamins.

7. The composition of claim 1 further comprising about 0.01 to 10 % by wt. of polyhydric alcohol(s) that are liquid or flowable at 25 C.

8. A method for preparing a stable cosmetic composition containing unsaturated fatty acid(s), comprising the steps of:
a. heating a non-aqueous silicone elastomer, silicone solvent and emulsifier blend to a temperature in the range of about 50-75 C until all the components are dissolved or well dispersed in a blend;
b. cooling the blend to a temperature lower than about 35C,
c. adding the conjugated fatty acid(s) to the blend with and/or followed by mixing; and
d. adding an aqueous solution of nitrogen containing borane(s) with and/or followed by mixing until the emulsion is uniform.

9. The method of claim 8 further comprising the step of adding an aqueous phase containing water soluble or dispersible neutralized alpha hydroxy acid(s) and/or vitamin(s) to the non-aqueous silicone elastomer, silicone solvent and emulsifier blend prepared in step (a) with and/or followed by mixing until the resulting emulsion is uniform and homogenous.

10. A method of removing malodorous decomposition products from a water in oil emulsion cosmetic composition containing conjugated fatty acid(s) comprising the step of adding a nitrogen containing borane compound; wherein the conjugated fatty acid(s) and nitrogen containing borane(s) are in the weight ratio range of about 15 to 1.

11. The method of claim 8 wherein the addition of the nitrogen containing borane compound is made after the composition has been stored at least for 30 days at a temperature of at least 20 C.

## Patentansprüche

1. Wasser-in-ÖI-Emulsions-Kosmetikzusammensetzung, umfassend:
a. etwa 0,01 bis 10 Gew.-% konjugierte Fettsäure(n),
b. etwa 0,01 bis 1 Gew.-% an Stickstoff enthaltendem(n) Boran(en), wobei das Gewichtsverhältnis der konjugierten Fettsäure(n) zu Stickstoff enthaltendem(n) Boran(en) in dem Bereich von etwa 15 bis 5 liegt und
c. etwa 0,5 bis 10 Gew.-% Emulgator(en) mit einem HLB-Wert in dem Bereich von etwa 2 bis 16.

2. Zusammensetzung nach Anspruch 1, wobei mindestens 10 Gew.-% der konjugierten Fettsäure(n) CLA (konjugierte Linolsäure) und/oder ein Derivat davon sind.

3. Zusammensetzung nach Anspruch 1, die ferner etwa 10 bis 70 Gew.-% Silikon-Elastomer(e) oder eine Mischung davon umfasst.

4. Zusammensetzung nach Anspruch 2, die ferner etwa 5 bis 20 Gew.-% Silikon-Lösungsmittel umfasst, das (die) zum Quellen des (der) Silikon-Elastomers (Silikon-Elastomere) fähig ist (sind), wobei das (die) Lösungsmittel bei 25°C flüssig oder fließfähig ist (sind).

5. Zusammensetzung nach Anspruch 1, die ferner etwa 0,01 bis 10 Gew.-% wasserlösliche oder dispergierbare Hautwirkstoffe umfasst.

6. Zusammensetzung nach Anspruch 5, wobei die Hautwirkstoffe alpha-Hydroxysäure(n) und/oder Vitamine einbeziehen.

7. Zusammensetzung nach Anspruch 1, die ferner etwa 0,01 bis 10 Gew.-% mehrwertigen Alkohol(e) umfasst, die bei 25°C flüssig oder fließfähig sind.

8. Verfahren zur Herstellung einer stabilen kosmetischen Zusammensetzung, die ungesättigte Fettsäure(n) enthält, umfassend die Schritte:
a. Erhitzen einer nicht-wässrigen Mischung von Silikon-Elastomer, Silikon-Lösungsmittel und Emulgator auf eine Temperatur in dem Bereich von etwa 50-75°C, bis sämtliche Komponenten gelöst oder in einer Mischung gut dispergiert sind,
b. Kühlen der Mischung auf eine Temperatur unter etwa 35°C,
c. Zugeben der konjugierten Fettsäure(n) zu der Mischung mit Mischen und/oder nachfolgendem Mischen und
d. Zugeben einer wässrigen Lösung von Stickstoff enthaltendem(n) Boran(en) mit Mischen und/oder nachfolgendem Mischen, bis die Emulsion gleichmäßig ist.

9. Verfahren nach Anspruch 8, das ferner den Schritt des Zugebens einer wässrigen Phase, die wasserlösliche oder dispergierbare neutralisierte alpha-Hydroxysäure(n) und/oder Vitamin(e) enthält, zu einer nicht-wässrigen Mischung von Silikon-Elastomer, Silikon-Lösungsmittel und Emulgator, hergestellt im Schritt (a), mit Mischen und/oder nachfolgendem Mischen, bis die anfallende Emulsion gleichmäßig und homogen ist.

10. Verfahren zum Entfernen von übelriechenden Abbauprodukten aus einer Wasser-in-ÖI-Emulsions-Kosmetikzusammensetzung, die konjugierte Fettsäure(n) enthält, umfassend den Schritt der Zugabe einer Stickstoff enthaltenden Boranverbindung, wobei die konjugierte Fettsäure(n) und das (die) Stickstoff enthaltende(n) Boran(e) in dem Gewichtsverhältnis des Bereichs von etwa 15 bis 1 liegen.

11. Verfahren nach Anspruch 8, wobei das Zugeben der Stickstoff enthaltenden Boranverbindung vorgenommen wird, nachdem die Zusammensetzung mindestens 30 Tage bei einer Temperatur von mindestens 20°C gelagert wurde.

## Revendications

1. Composition cosmétique d'émulsion eau-dans-huile, comprenant :
a. environ 0,01 à 10 % en masse d'acide(s) gras conjugué(s) ;
b. environ 0,01 à 1 % en masse de borane(s) contenant de l'azote, où le rapport massique du(des) acide(s) gras conjugué(s) à borane(s) contenant de l'azote se trouve dans l'intervalle d'environ 15 à 5 ; et
c. environ 0,5 à 10 % en masse d'émulsionnant(s) ayant une valeur HLB dans l'intervalle d'environ 2 à 16.

2. Composition selon la revendication 1, où au moins 10 % en masse du(des) acide(s) gras conjugué(s) est CLA (acide linoléique conjugué) et/ou un dérivé de celui-ci.

3. Composition selon la revendication 1 comprenant de plus environ 10 à 70 % en masse d'élastomère(s) de silicone ou une combinaison de ceux-ci.

4. Composition selon la revendication 2 comprenant de plus d'environ 5 à 20 % en masse de solvant(s) de silicone qui est(sont) capable(s) de gonfler le(les) élastomère(s) de silicone où le(les) solvant(s) est(sont) liquide(s) ou fluide(s) à 25°C.

5. Composition selon la revendication 1 comprenant de plus d'environ 0,01 à 10 % en masse d'actifs pour la peau dispersibles ou solubles dans l'eau.

6. Composition selon la revendication 5, où les actifs pour la peau comprennent du(des) acide(s) alpha hydroxy et/ou des vitamines.

7. Composition selon la revendication 1 comprenant de plus d'environ 0,01 à 10 % en masse d'alcool(s) polyvalent(s) qui est(sont) liquide(s) ou fluide(s) à 25°C.

8. Procédé de préparation d'une composition cosmétique stable contenant un(des) acide(s) gras insaturé(s), comprenant les étapes de :
a. chauffage d'une combinaison d'élastomère de silicone, de solvant de silicone et d'émulsionnant non aqueuse à une température dans l'intervalle d'environ 50-75°C jusqu'à ce que tous les constituants soient dissous ou bien dispersés dans une combinaison ;
b. refroidissement de la combinaison à une température inférieure à environ 35°C,
c. addition du(des) acide(s) gras conjugué(s) à la combinaison avec et/ou mélange subséquent ; et
d. addition d'une solution aqueuse de borane(s) contenant de l'azote avec et/ou mélange subséquent jusqu'à ce que l'émulsion soit uniforme.

9. Procédé selon la revendication 8 comprenant de plus l'étape d'addition d'un(d') acide(s) alpha hydroxy neutralisé(s) dispersible(s) ou soluble(s) dans l'eau contenant une phase aqueuse et/ou une(des) vitamine(s) à la combinaison d'élastomère de silicone, de solvant de silicone et d'émulsionnant non aqueuse préparée dans l'étape (a) avec et/ou mélange subséquent jusqu'à ce que l'émulsion résultante soit uniforme et homogène.

10. Procédé d'élimination de produits de décomposition mal odorants d'une composition cosmétique d'émulsion eau-dans-huile contenant un(des) acide(s) gras conjugué(s) comprenant l'étape d'addition d'un composé de borane contenant de l'azote ; où l'(les) acide(s) gras conjugué(s) et le(les) borane(s) contenant de l'azote sont dans l'intervalle de rapport massique d'environ 15 à 1.

11. Procédé selon la revendication 8, où l'addition du composé de borane contenant de l'azote est réalisée après que la composition a été stockée au moins pendant 30 jours à une température d'au moins 20°C.
